# EUROPÄISCHE PATENTSCHRIFT

(11) **EP 2 049 894 B1**
(45) Veröffentlichungstag und Bekanntmachung des Hinweises auf die Patenterteilung: **19.01.2011**
(21) Anmeldenummer: 07785921.3
(22) Anmeldetag: 06.07.2007
(51) Int. Cl.: G01N 33/487

(54) **VORRICHTUNG UND VERFAHREN ZUM ERFASSEN VON PARTIKELN MIT PIPETTE UND NANOPORE**
DEVICE AND METHOD FOR DETECTING ARTICLES WITH PIPETTE AND NANOPORE
DISPOSITIF ET PROCÉDÉ DE DÉTECTION DE PARTICULES AVEC UNE PIPETTE ET DES NANOPORES

(30) Priorität: 28.07.2006 DE 102006035072
(43) Veröffentlichungstag der Anmeldung: 22.04.2009
(73) Patentinhaber: Westfälische Wilhelms-Universität Münster, 48149 Münster (DE)
(72) Erfinder: SCHÄFFER, Tilman, 91052 Erlangen (DE); FUCHS, Harald, 48149 Münster (DE)
(74) Vertreter: Lucke, Andreas
(86) Internationale Anmeldenummer: PCT/EP2007/006012
(87) Internationale Veröffentlichungsnummer: WO 2008/011972

(56) Entgegenhaltungen:
- EP-A- 1 225 234
- EP-A- 1 635 160
- EP-A- 1 645 628

## Beschreibung

Die Erfindung betrifft eine Vorrichtung zum Erfassen und/oder Charakterisieren von Partikeln, die folgendes umfaßt: mindestens eine Nanopore, eine Spannungsquelle zum Erzeugen eines elektrischen Potentialunterschieds zwischen den beiden Seiten der mindestens einen Nanopore, um einen Ionenstrom durch die Nanopore zu erzeugen, wenn die Nanopore von einem Elektrolyten umgeben ist, und eine Meßeinrichtung, die geeignet ist, eine Änderung der Impedanz der mindestens einen Nanopore bezüglich des Ionenstroms zu erfassen, wenn eines oder mehrere von zu erfassenden Partikeln, die in dem Elektrolyt enthalten sind, die mindestens eine Nanopore durchquert bzw. durchqueren. Ferner betrifft die Erfindung ein Verfahren zum Erfassen und/oder Charakterisieren von Partikeln nach dem Oberbegriff des Anspruchs 21.

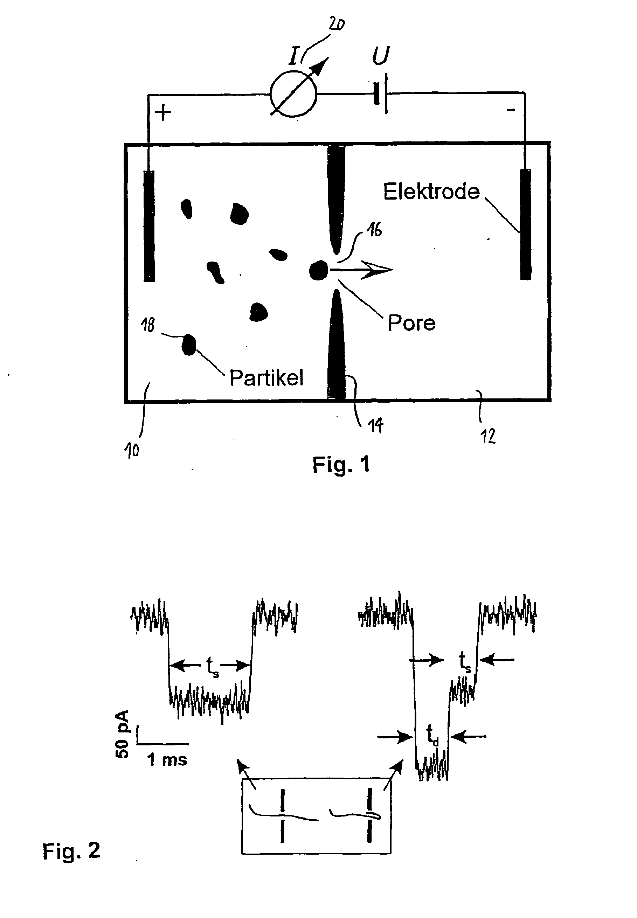

Nanoporen sind Poren mit Durchmessern im Nanometerbereich. Nanoporen werden zunehmend für die Detektion, Analyse und Sequenzierung von Einzelmolekülen verwendet, wie dies beispielsweise in Nakane, J.J., et al. "Nanopore sensors for nucleic acid analysis" J. Phys.: Condens. Matter 15: R1365-R1393 beschrieben ist. Mit Hilfe von Nanoporen ist es möglich, Informationen über die Zusammensetzung und Struktur der untersuchten Partikeln zu erhalten, bei denen es sich typischerweise um Moleküle handelt. Eine Vorrichtung der eingangs genannten Art ist in Fig. 1 schematisch dargestellt. Die Vorrichtung von Fig. 1 umfaßt zwei Kammern 10, 12, die durch eine Membran 14 getrennt sind. In der Membran ist eine Nanopore 16 ausgebildet. In beiden Kammern 10 und 12 befindet sich ein Elektrolyt. In der in der Darstellung von Fig. 1 linken Kammer 10 befinden sich außerdem Partikel 18. Mit dem Begriff "Partikel" werden in der vorliegenden Schrift sämtliche kleinen Materialmengen bezeichnet, die dem beschriebenen Verfahren zum Erfassen und/oder Charakterisieren prinzipiell zugänglich sind, insbesondere Moleküle, Molekülkomplexe, Makromoleküle wie Proteine und Nukleinsäuren etc.

Die Nanopore 16 bildet den einzigen Durchgang für den Elektrolyten und die Partikel 18 zwischen den Kammern 10 und 12. Wird nun eine Spannung zwischen den Kammern 10, 12 angelegt, fließt ein Ionenstrom durch die mit dem Elektrolyten gefüllte Pore 16. Wenn die Partikel 18 geladen sind, werden sie ebenfalls elektrophoretisch durch die Nanopore 16 getrieben. Falls die Partikel 18 ungeladen sind, können sie durch einen Druckgradienten oder durch elektroosmotischen Fluß durch die Pore getrieben werden.

Bei der Vorrichtung von Fig. 1 wird der Ionenstrom durch die Nanopore 16 mit einer Strommeßvorrichtung 20 gemessen. Beim Durchgang eines oder mehrerer Partikel 18 durch die Nanopore 16 wird diese kurzzeitig blockiert, was zu einem vorübergehenden Abfall im gemessenen Ionenstrom führt. Durch die Häufigkeit, Dauer und Form des Abfalls läßt sich auf die Konzentration und die Eigenschaften (z.B. Größe und Aufbau) der Partikel schließen. Die Strommeßvorrichtung 20 ist somit ein Beispiel für eine Meßeinrichtung, die geeignet ist, eine Änderung der Impedanz der Nanopore 16 bezüglich des Ionenstroms zu erfassen, wenn eines oder mehrere von den zu erfassenden Partikeln 18 die Nanopore 16 durchquert.

In Fig. 2 sind Stromsignale gezeigt, die beim Durchgang von Partikeln (hier DNA) durch die Nanopore 16 erhalten werden. Die Messungen sind aus Chen, P., et al. (2004), "Probing Single DNA Molecule Transport Using Fabricated Nanopores", Nano Lett. 4 (11): 2293-2298 entnommen. Das linke Stromsignal zeigt einen Stromabfall im Millisekundenbereich, wenn ein DNA-Molekül "gestreckt" durch die Nanopore 16 tritt. Das rechte Signal zeigt einen Fall, in dem das DNA-Molekül im teilweise gefalteten Zustand durch die Nanopore 16 tritt. Wie im rechten Stromsignal zu erkennen ist, fällt der Strom während des Durchgangs des DNA-Moleküls auf zwei unterschiedlich tiefe Niveaus ab, die für den Grad der Blockierung der Nanopore 16 kennzeichnend sind.

Die in Fig. 1 schematisch gezeigte Vorrichtung ist unter dem Namen "Coulter"-Zähler bekannt und beispielsweise in Bayley, H. und C.R. Martin (2000), Chem.Rev. 100: 2575-2594 beschrieben.

Der bekannte Coulter-Zähler von Fig. 1 wird üblicherweise für quantitative Messungen von Größen und Konzentrationen von Makromolekülen in einem Reservoir verwendet, das in der Darstellung von Fig. 1 durch die linke Kammer 10 gebildet wird. Eine weitere Anwendung, an der gegenwärtig gearbeitet wird, besteht darin, DNA-Sequenzen mit Hilfe des Coulter-Zählers zu entschlüsseln. Da das Ausmaß des Stromabfalls ein Maß für die physikalische Größe desjenigen Teils des DNA-Moleküls ist, welches sich im engsten Teil der Pore 16 befindet, und da die Nukleotide des DNA-Moleküls leicht unterschiedliche Größen haben, sollte man in der Lage sein, die genetische Information der Nukleotide eines DNA-Moleküls durch zeitaufgelöste Messung des Ionenstroms durch die Nanopore, d.h. die zeitabhängige Variation der Impedanz der Nanopore 16 beim Durchtritts eines DNA-Moleküls zu entschlüsseln. Gegenwärtige Anwendungen des Coulters-Zählers von Fig. 1 beschränken sich also auf die Analyse von Partikeln, die ungeordnet in einem Reservoir enthalten sind.

Die EP 1 225 234 A2 offenbart ein Verfahren zum Sequenzieren von DNA-Molekülen nach dem oben skizzierten Verfahren, bei dem zwei Lösungen in zwei benachbarten Reservoiren vorgesehen sind, die lediglich durch einen engen Kanal, d.h. eine Nanopore verbunden sind. Die Besonderheit bei dieser besteht darin, dass das DNA-Molekül modifizierte Nukleotide enthält, die die Sekundärstruktur in dem DNA-Molekül verringert, wodurch die Sequenzierungsrate erhöht werden kann. Die zu sequenzierende DNA befindet sich ungeordnet in dem Ausgangsreservoir. In einer Ausführungsform schlägt diese Schrift vor, ein Reservoir durch den Innenraum einer Pipette zu bilden, an deren Öffnung eine Lipid-Doppelschicht angeordnet ist, in der eine Nanopore ausgebildet ist.

Der vorliegenden Erfindung liegt die Aufgabe zugrunde, die Vorrichtung und das Verfahren der eingangs genannten Art weiterzuentwickeln, um sie bzw. es für weitere Anwendungen, jenseits der oben beschriebenen Analyse von Partikeln nutzbar zu machen.

Diese Aufgabe wird bei der Vorrichtung der eingangs genannten Art dadurch gelöst, daß die Vorrichtung eine Pipette mit einem Endabschnitt umfaßt, in dem eine Öffnung ausgebildet ist, daß sie Mittel zum Erzeugen eines Partikelflusses von den zu erfassenden Partikeln von außerhalb der Pipette durch die Öffnung und durch die Nanopore umfaßt und daß sie einen XYZ-Scanner zum Bewegen der Pipette relativ zu einer Probe umfaßt.

Ferner wird die Aufgabe durch ein Verfahren nach Anspruch 12 gelöst.

Bei der erfindungsgemäßen Vorrichtung werden somit die zu erfassenden Partikel durch die Öffnung im Endabschnitt der Pipette aufgenommen. Da die Pipette relativ zu einer Probe bewegt werden kann, können mit Hilfe der erfindungsgemäßen Vorrichtungen Partikel ortsaufgelöst erfaßt und/oder charakterisiert werden, wie unten anhand von Ausführungsbeispielen näher erläutert wird.

Außerdem ermöglicht es die Pipette die erfaßten und/oder charakterisierten Partikel "einzufangen". Dadurch können einmal gemessene Partikel aufbewahrt und gegebenenfalls noch einmal gemessen werden. Dies ist ein wesentlicher Unterschied zu dem Coulter-Zähler von Fig. 1, bei dem Partikel 18, die von der linken Kammer 10 durch die Nanopore 16 in die rechte Kammer 12 gelangen, ungehindert durch die gesamte rechte Kammer 12 diffundieren und nicht gezielt weiteren Messungen unterzogen werden können.

Die Mittel zum Erzeugen des Partikelflusses können zumindest teilweise durch die genannte Spannungsquelle gebildet werden. Dies bietet sich insbesondere dann an, wenn die zu erfassenden Partikel geladen sind und ähnlich wie die Ionen des Elektrolyten durch Elektrophorese in die Pipette hinein und durch die Nanopore getrieben werden. Jedoch kann auch bei ungeladenen Partikeln ein Partikelfluß als sogenannter elektroosmotischer Fluß in Folge des elektrischen Potentialunterschiedes zwischen den beiden Seiten der mindestens einen Nanopore erzeugt werden.

Falls die zu erfassenden Partikel nicht geladen sind, umfaßt die Vorrichtung vorzugsweise Mittel zum Erzeugen eines Druckunterschiedes zwischen einem Abschnitt im Inneren und einem Abschnitt im Äußeren der Pipette.

Vorzugsweise hat die mindestens eine Nanopore eine Öffnungsfläche von weniger als 2000 nm², besonders vorzugsweise von weniger als 700 nm² und insbesondere von weniger als 200 nm². In einer vorteilhaften Weiterbildung hat die mindestens eine Nanopore eine veränderbare Größe. Die Nanopore veränderbarer Größe gestattet es, die Vorrichtung für das Erfassen und/oder Charakterisieren von Partikeln unterschiedlicher Größe zu verwenden. Durch Ändern der Größe der Nanopore ist es außerdem möglich, die Partikel nach ihrer Größe zu diskriminieren und sogar zu sortieren. Auch kann die Größe der Nanopore in Abhängigkeit von den Eigenschaften der zu erfassenden Teilchen so eingestellt werden, daß sich ein optimales Signal-zu-Rauschen-Verhältnis ergibt.

In einer vorteilhaften Ausführungsform wird die mindestens eine Nanopore veränderbarer Größe durch einen Kanal in einem flexiblen Material gebildet, dessen Querschnitt durch Druck auf das flexible Material verengbar ist. In einer anderen vorteilhaften Ausführungsform ist die mindestens eine Nanopore in einer Membran angeordnet und der Querschnitt der Nanopore durch Dehnung der Membran veränderbar.

Vorzugsweise umfaßt die Vorrichtung einen Probenbehälter zum Aufnehmen des Elektrolyten und Bewegungsmittel zum Bewegen der Pipette und des Probenbehälters relativ zueinander. Der Begriff "Probe" bezeichnet in der vorliegenden Schrift allgemein jeden Gegenstand oder jedes Objekt, das mit der Vorrichtung und dem Verfahren der Erfindung untersucht werden kann. Beispielsweise kann eine Probe einfach durch einen Elektrolyten gebildet werden, in dem sich zu erfassende Partikel befinden, oder durch ein biologisches System wie beispielsweise lebende Zellen, die von einem Elektrolyten umgeben sind.

In einer vorteilhaften Weiterbildung sind die Bewegungsmittel dazu ausgelegt, den Endabschnitt der Pipette derart in ein der Öffnung im Endabschnitt gegenüberliegendes nachgiebiges Material zu drücken, daß sich die effektive Öffnungsfläche der Öffnung verringert, um dadurch eine Nanopore veränderbarer Größe zu bilden. Gemäß dieser Weiterbildung wird die Nanopore also dadurch gebildet, daß die Öffnung im Endabschnitt der Pipette teilweise verschlossen bzw. zugedeckt wird, wenn der Endabschnitt der Pipette in das nachgiebige Material gedrückt wird. In einer vorteilhaften Ausführungsform kann das nachgiebige Material durch eine Fläche aus einem Polymermaterial, insbesondere aus Poly-(dimethylsiloxan) gebildet werden. Das nachgiebige Material kann aber auch durch die Probe selbst, beispielsweise eine Zelle gebildet werden, in die der Endabschnitt der Pipette gedrückt wird.

Die Vorrichtung umfasst ferner vorzugsweise eine Steuerungseinrichtung zum Ansteuern des XYZ-Scanners zum Abtasten der Oberfläche einer Probe, in dem der Endabschnitt der Pipette in einem konstanten Abstand über die Oberfläche der Probe gefahren wird. Gemäß dieser vorteilhaften Ausführungsform wird die Pipette somit zu einem Rastersondenmikroskop weitergebildet. Dadurch kann eine Probe abgetastet werden, und gleichzeitig können die Partikel ortsaufgelöst durch die Öffnung der Pipette aufgenommen und mit Hilfe der Nanopore erfaßt bzw. charakterisiert werden.

Vorzugsweise umfaßt die Vorrichtung eine Datenverarbeitungseinrichtung, die so programmiert ist, daß sie die beim Abtasten der Oberfläche der Probe durchgeführten Relativbewegungen zwischen dem Endabschnitt der Pipette und der Probe aufzeichnet und daraus ein topographisches Bild der Probe und/oder ein Bild erzeugt, das die Messungen der Partikel in Abhängigkeit vom Ort der Pipettenöffnung repräsentiert.

In einer vorteilhaften Ausführungsform wird der Abstand zwischen dem Endabschnitt der Pipette und der Probe basierend auf einem Ionenstrom durch die Öffnung im Endabschnitt der Pipette eingestellt. Gemäß dieser Weiterbildung nimmt die Vorrichtung zusätzlich die Funktion eines Raster-Ionenleitfähigkeits-Mikroskops an, wie unten anhand eines Ausführungsbeispiels erläutert wird. In einer anderen Ausführungsform kann der Abstand zwischen dem Endabschnitt der Pipette und der Probe basierend auf Scherkräften eingestellt werden, die auf den Endabschnitt der Pipette wirken, wie ebenfalls unten anhand eines Ausführungsbeispiels näher erläutert wird.

Vorzugsweise hat der Innenraum der Pipette eine erste Kammer, die direkt mit der Öffnung im Endabschnitt der Pipette verbunden ist, und eine zweite Kammer, die von der ersten Kammer durch eine Trennwand getrennt ist, in welcher die mindestens eine Nanopore ausgebildet ist. Vorzugsweise enthält die Pipette ferner eine dritte Kammer, die von der ersten Kammer durch eine Trennwand getrennt ist, in welcher ebenfalls mindestens eine Nanopore ausgebildet ist. Dabei haben die Nanopore in der Trennwand zwischen der ersten Kammer und der zweiten Kammer und die Nanopore in der Trennwand zwischen der ersten Kammer und der dritten Kammer vorzugsweise eine unterschiedliche Öffnungsfläche. Dies ermöglicht es beispielsweise, zu erfassende Partikel nach ihrer Größe zu sortieren und/oder Messungen an Partikeln unterschiedlicher Größe simultan durchzuführen.

Zusätzlich oder alternativ hat die Differenz zwischen den elektrischen Potentialen der zweiten Kammer und der ersten Kammer ein anderes Vorzeichen als die Differenz zwischen den elektrischen Potentialen der dritten Kammer und der ersten Kammer. Dadurch können die zu erfassenden Partikel auch ihrer Ladung nach differenziert und sortiert werden.

Weitere Vorteile und Merkmale der vorliegenden Erfindung ergeben sich aus der nachfolgenden Beschreibung, in der die Erfindung anhand mehrerer Ausführungsbeispiele unter Bezugnahme auf die beigefügten Zeichnungen erläutert wird, von denen
- Fig. 1: eine schematische Darstellung einer Vorrichtung zum Erfassen und/oder Charakteri- sieren von Partikeln nach dem Stand der Technik zeigt;
- Fig. 2: Stromsignale zeigt, die eine Änderung der Impedanz einer Nanopore bezüglich eines Ionenstroms charakterisieren, wenn ein Partikel eine Nanopore durchquert;
- Fig. 3: eine schematische geschnittene Darstellung einer Vorrichtung gemäß einer Weiterbil- dung der Erfindung zeigt;
- Fig. 4 und 5: das Verhältnis zwischen dem Ionenstrom und dem Abstand zwischen einer Pipettenöffnung und einer Probe zeigen;
- Fig. 6: eine Schnittansicht einer Vorrichtung nach einer anderen Weiterbildung der Erfindung zeigt;
- Fig. 7 bis 9: unterschiedliche Ausführungsformen von Pipetten für Vorrichtungen nach Weiterbildungen der Erfindung zeigen;
- Fig. 10: eine schematische Darstellung von Momentaufnahmen beim Ausbilden einer Nanopo- re veränderlicher Größe zeigt, indem der Endabschnitt einer Pipette in ein nachgiebi- ges Material gedrückt wird; und
- Fig. 11 und 12: Beispiele für Nanoporen unterschiedlicher Größe.

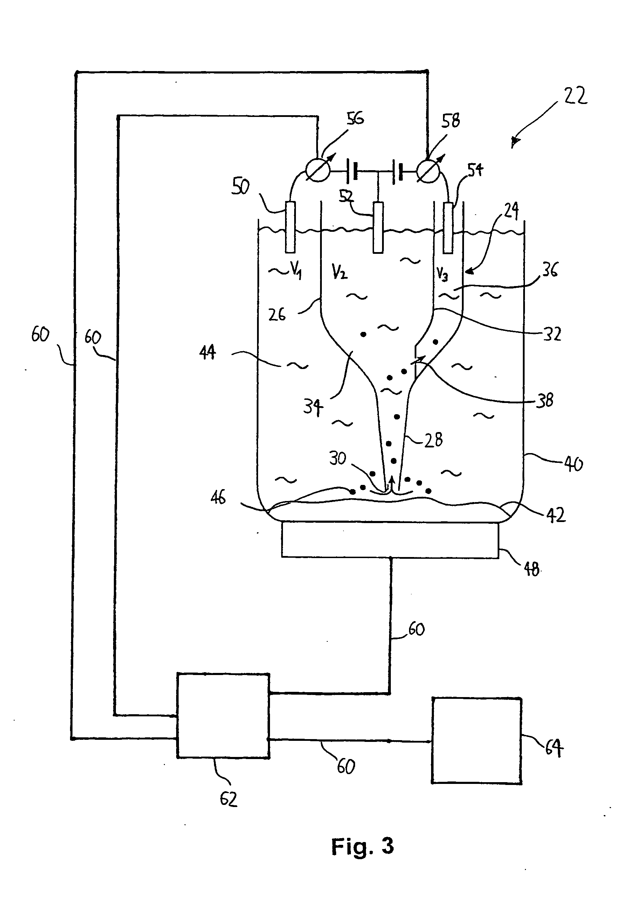

In Fig. 3 ist eine Vorrichtung 22 zum Erfassen oder Charakterisieren von Partikeln schematisch dargestellt. Die Vorrichtung 22 umfaßt eine Pipette 24 mit einem Körperabschnitt 26 und einem Endabschnitt 28, in dem eine Öffnung 30 ausgebildet ist. Die Pipette 24 wird auch als "Nanopipette" bezeichnet. Sie ist beispielsweise aus einem Glas-Röhrchen mit einem Innendurchmesser von typischerweise 1 bis 2 mm hergestellt, dessen eines Ende erhitzt und langgezogen wird, um den Endabschnitt 28 zu bilden. In dem ausgezogenen Endabschnitt 28 verringert sich der Innendurchmesser der Pipette 24 über eine Strecke von typischerweise einigen Millimetern auf einen minimalen Durchmesser bei der Öffnung 30, der zwischen 40 nm und einigen Mikrometern betragen kann.

Im Körperabschnitt 26 der Pipette 24 ist eine Trennwand 32 ausgebildet. Die Trennwand 32 trennt eine erste Kammer 34 des Innenraums der Pipette 24, die mit der Öffnung 30 verbunden ist, von einer zweiten Kammer 36. In der Trennwand 32 ist eine Nanopore 38 ausgebildet, die die einzige Verbindung zwischen der ersten und der zweiten Kammer 34, 36 bildet.

Die Nanopore 38 hat einen Querschnitt bzw. eine Öffnungsfläche, die so gewählt ist, daß sie sich zum Erfassen und Charakterisieren von Partikeln nach dem Coulter-Prinzip eignet, welches im Zusammenhang mit Fig. 1 beschrieben wurde. Der Durchmesser der Nanopore 38 hängt in der konkreten Anwendung daher von der Größe der Partikel ab, die zu detektieren sind. In einer typischen Anwendung ist der Durchmesser der Nanopore 38 kleiner als 30 nm (entsprechend einer Öffnungsfläche von ca. 700 nm²) und vorzugsweise kleiner als 15 nm (entsprechend einer Öffnungsfläche von rund 200 nm²). Bei dieser Gelegenheit wird darauf hingewiesen, daß bei der Pipette 24 in manchen Ausführungsformen die Öffnung 30 selbst im Nanometerbereich liegt und daher als "Nanopore" bezeichnet werden könnte. Jedoch ist die Öffnung 30 im gezeigten Ausführungsbeispiel deutlich größer als die Nanopore 38, und sie erfüllt auch nicht die Funktion einer Nanopore im Sinne des Coulter-Prinzips. Wenn nämlich der Endabschnitt 28 der Pipette 24 so stark ausgezogen wäre, daß die Öffnung 30 beispielsweise auf einen Durchmesser von einigen Nanometern verengt würde, wäre der Widerstand für den Ionenfluß durch den Endabschnitt 28, d.h. den Halsabschnitt der Pipette 24, zu hoch, als daß sich mit den gegenwärtig bekannten Meßvorrichtungen Coulter-Messungen guter Qualität erreichen ließen.

Die Pipette 24 ist in einen Behälter 40 getaucht, auf dessen Boden sich eine Probe 42 befindet, bei der es sich beispielsweise um lebende Zellen handelt. Der Behälter 40 ist mit einem Elektrolyten 44 gefüllt, der in der Darstellung von Fig. 3 durch Wellensymbole angedeutet ist. In dem Elektrolyten 44 befinden sich ferner Partikel 46, beispielsweise Moleküle oder Molekülkomplexe, die in Fig. 3 durch Punkte dargestellt sind, von denen eines exemplarisch mit Bezugzeichen 46 bezeichnet ist.

Das Gefäß 40 ist auf einem XYZ-Scanner 48 angeordnet, mit dem es relativ zur Pipette 24 in allen drei Raumrichtungen bewegt werden kann. Alternativ könnte auch ein XYZ-Scanner vorgesehen sein, mit dem die Pipette 24 relativ zum Gefäß 40 und der darin enthaltenen Probe 42 bewegt würde.

Eine erste Elektrode 50 ist außerhalb der Pipette 24 in den Elektrolyten 44 getaucht und hält den Elektrolyten 44 in dem Gefäß 40 auf einem ersten Potential V₁. In der ersten Kammer 34 der Pipette 24 befindet sich eine zweite Elektrode 52, die den Elektrolyten in der ersten Kammer 34 auf einem Potential V₂ hält. Schließlich befindet sich in der zweiten Kammer 36 eine dritte Elektrode 54, die den Elektrolyten in der zweiten Kammer 36 auf einem dritten Potential V₃ hält. Eine erste Strommeßvorrichtung 56 mißt im wesentlichen (d.h. bis auf den Strom, der durch die Nanopore in die zweite Kammer 36 fließt) den Ionenstrom des Elektrolyten zwischen dem Behälter 40 und der ersten Kammer 34. Eine zweite Strommeßvorrichtung 58 mißt den Ionenstroms des Elektrolyten zwischen der ersten Kammer 34 und der zweiten Kammer 36. Die von den Strommeßvorrichtungen 56 und 58 gemessenen Ströme werden über Signalleitungen 60 zu einem Computer 62 übertragen. Der Computer 62 ist über eine weitere Signalleitung 60 mit dem XYZ-Scanner 48 verbunden, über die er diesen ansteuern kann. Ferner ist der Computer 62 über eine weitere Signalleitung 60 mit einem Ausgabegerät, beispielsweise einem Bildschirm verbunden.

Im folgenden wird die Funktion der Vorrichtung 22 von Fig. 3 beschrieben. Der Computer 62 steuert den XYZ-Scanner 48 derart an, daß der Endabschnitt 28 der Pipette 24 in einem konstanten Abstand über die Oberfläche der Probe gefahren wird. Der Abstand zwischen dem Endabschnitt der Pipette 24 und der Probe 42 wird dabei nach einem Prinzip eingestellt, welches per se von sogenannten Raster-Ionenleitfähigkeits-Mikroskopen bekannt ist. Aufgrund des Unterschiedes zwischen den Potentialen V₁ und V₂ fließt ein Ionenstrom zwischen dem Behälter 40 und der ersten Kammer 34. Dieser Ionenstrom wird mit der Strommeßvorrichtung 56 gemessen. Wenn sich die Öffnung 30 der Probe 42 nähert, tritt ab einer bestimmten Entfernung ein sogenannter "Abschnüreffekt" auf, aufgrund dessen der Durchtritt von Ionen durch die Öffnung 30 erschwert wird, was zu einem Abfall des mit der Vorrichtung 56 gemessenen Ionenstroms führt.

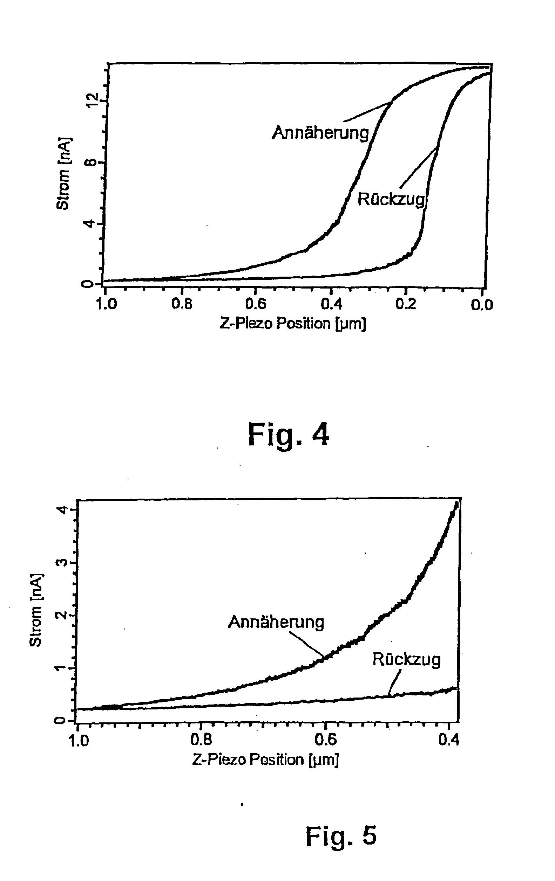

Fig. 4 und 5 zeigen das Verhältnis zwischen dem Abstand zwischen dem Endabschnitt 28 der Pipette 24 und der Probe 42 einerseits und dem gemessenen Ionenstrom durch die Öffnung 30 andererseits. In den Diagrammen von Fig. 4 und 5 ist auf der Abszisse die vertikale Bewegung (d.h. Bewegung in Z-Richtung) des Behälters 40 durch den XYZ-Scanner aufgetragen und auf der Ordinate der mit der Strommeßvorrichtung 56 gemessene Strom. Fig. 5 zeigt einen vergrößerten Ausschnitt der Messung von Fig. 4. In beiden Diagrammen ist die Strom-Abstandskurve sowohl für das Annähern der Pipette 24 an die Probe als auch für deren Zurückziehen gezeigt. Die Abweichung der beiden Kurven voneinander demonstriert eine Hysterese des beschriebenen "Abschnüreffekts".

In einer Ausführungsform wird die Pipette 24 so über die Probe 42 gefahren, daß der mit der Strommeßvorrichtung 56 gemessene Strom auf einem konstanten Wert gehalten wird. Der gemessene Strom entspricht nahezu dem Ionenstrom durch die Öffnung 30, vermindert um den Ionenstrom durch die Nanopore 38, der aber aufgrund der geringen Größe der Nanopore 38 nur einen kleineren Anteil ausmacht. Der Computer 62 steuert den XYZ-Scanner so an, daß der Ionenstrom und damit der Abstand zwischen Pipette 24 und Proben 42 beim Abrastem konstant gehalten wird. Die beim Abtasten der Oberfläche der Probe 42 durchgeführten Bewegungen des XYZ-Scanners werden vom Computer 62 aufgezeichnet, und aus ihnen wird ein topographisches Bild der Oberfläche erzeugt, welches an die Ausgabevorrichtung 64 ausgegeben wird. Alternativ zum beschriebenen Verfahren könnte die Pipette 24 auch zumindest abschnittsweise auf einem konstanten horizontalen Niveau (d.h. bei einen konstanten Z-Wert des XYZ-Scanners 48) über die Probe 42 gefahren werden und der Abstand zwischen der Öffnung 30 und der Probe 42 durch Änderungen im gemessenen Strom unter Zuhilfenahme der Diagramme von Fig. 4 und 5 gemessen werden, um dadurch ebenfalls ein topographisches Bild bzw. Relief der Probe 42 zu erzeugen.

In der Ausführungsform von Fig. 3 sind die zu erfassenden Partikel 46 geladen, und sie werden aufgrund der Unterschiede zwischen den Potentialen V₁, V₂ und V₃ von außerhalb der Pipette 24 durch die Öffnung 30 und durch die Nanopore 38 in die zweite Kammer 36 bewegt. Beim Durchtritt der Partikel 46 durch die Nanopore 38 wird die Impedanz der Nanopore 38 bezüglich des Ionenstroms des Elektrolyten variiert, was bei der Strommeßvorrichtung 58 zu Stromsignalen von der Art führt, wie sie in Fig. 2 gezeigt sind. Durch Analyse der Stromsignale der Strommeßvorrichtung 58 können die Partikel 46 auf ähnliche Weise erfaßt und charakterisiert werden, wie dies im Zusammenhang mit dem Coulter-Zähler von Fig. 1 erläutert wurde.

Es wird jedoch deutlich, daß die Funktion der Vorrichtung von Fig. 3 weit über diejenige des herkömmlichen Coulter-Zählers von Fig. 1 hinausgeht. Da der Partikelfluß der Partikel 46 durch die Öffnung 30 der Pipette 24 geht, können die Partikel je nach gegenwärtiger Position der Pipette 24 bezüglich der Probe 42 ortsaufgelöst erfaßt und charakterisiert werden. Wenn die Probe 42 beispielsweise eine lebende Zelle ist, in deren Zellmembran Kanäle ausgebildet sind, durch die Partikel 46 hindurchtreten, können die austretenden Partikel ortsaufgelöst detektiert werden. Z.B. können die Kanäle durch eine lokal erhöhte Konzentration der Partikel "sichtbar" gemacht werden. Auch können Partikel, die durch Öffnungen zwischen Zellen einer Zellschicht hindurchtreten, erfaßt und charakterisiert werden. Ähnliche Untersuchungen können bisher nur durch Markierung von Molekülen durchgeführt werden. Die Vorrichtung von Fig. 3 hingegen gestattet eine markierungsfreie Detektion von Molekülen, insbesondere Biomolekülen. Auch kann durch Analyse der Stromsignale an der Strommeßvorrichtung 58 die Größe, Zusammensetzung und/oder Struktur der Partikel analysiert werden.

Ein weiterer funktioneller Unterschied zwischen der Vorrichtung von Fig. 3 und einem herkömmlichen Coulter-Zähler besteht darin, daß die Partikel nach dem Durchtritt durch die Nanopore 38 nicht unkontrolliert durch ein großes Reservoir diffundieren. Statt dessen befinden sich die Partikel 46 nach dem Durchtritt durch die Nanopore 38 in der räumlich eng begrenzten zweiten Kammer 36, und sie können aus dieser zur weiteren Analyse entnommen werden. Auch besteht die Möglichkeit, ein Partikel durch Umpolen der Spannung zwischen den Elektroden 52 und 54 mindestens zweimal durch die Nanopore 38 hin und her zu treiben, wodurch weitere Information über das Partikel gewonnen werden kann. Allgemein gestattet der Aufbau mit der Pipette 24 und insbesondere mit deren Kammern es, die zu untersuchenden oder bereits untersuchten Partikel einzuschließen, um sie für weitere Untersuchungen verfügbar zu machen.

Die von der Strommeßvorrichtung 58 gemessenen Signale werden vom Computer 62 verarbeitet. Der Computer 62 kann ein Bild erzeugen, das die Messungen der Partikel, beispielsweise deren Konzentration, in Abhängigkeit vom Ort der Pipettenöffnung 30 repräsentiert.

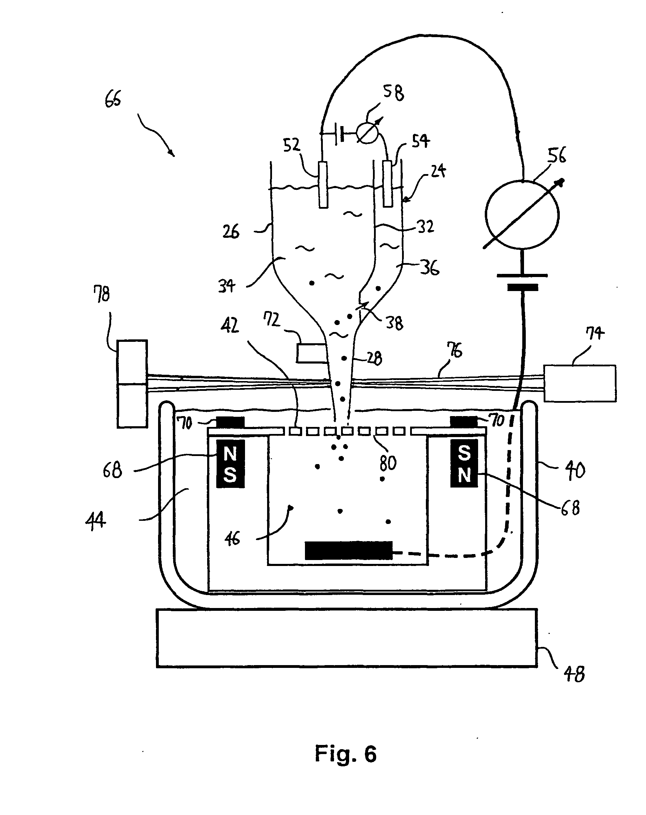

In Fig. 6 ist eine alternative Ausführungsform einer Vorrichtung 66 nach einer Weiterbildung der Erfindung gezeigt. Gleiche oder ähnliche Bestandteile der Vorrichtung 66 werden mit denselben Bezugszeichen bezeichnet wie in Fig. 3. Die Vorrichtung 66 umfaßt eine Pipette 24, deren Aufbau mit derjenigen von Fig. 3 identisch ist und daher nicht noch einmal beschrieben wird. Die Vorrichtung 66 umfaßt ebenfalls einen Behälter 40, in dem ein Elektrolyt 44 und zu erfassende Partikel 46 enthalten sind, eine Probe 42, die im Ausführungsbeispiel von Fig. 6 durch eine biologische Membran gebildet ist, und einen XYZ-Scanner 48. Die Membran 42 ist zwischen Magneten 68 und einem Eisenring 70 eingespannt und wird dadurch gehalten.

Der Hauptunterschied zwischen den Vorrichtungen von Fig. 3 und 6 besteht in der Abstandsmessung zwischen dem Endabschnitt 28 der Pipette 24 und der Probe 42. Bei der Vorrichtung 66 von Fig. 6 wird der Abstand durch Scherkräfte bestimmt, die auftreten, wenn sich der Endabschnitt der Pipette 24 der Probe 42 nähert. Dazu wird der Endabschnitt 28 der Pipette 24 mit einem Piezoaktor 72 in Schwingungen versetzt. Ein Laser 74 erzeugt einen Laserstrahl 76, der auf den Endabschnitt 28 der Pipette 24 fokussiert ist. Der Laserstrahl 76 trifft auf einen positionsempfindlichen Sensor, beispielsweise einen segmentierten Fotodetektor 78. Wenn sich der Endabschnitt 28 der Pipette 24 der Probe 42 nähert, treten Scherkräfte auf, die die Vibration des Endabschnitts 28 dämpfen. Die Modulation der Vibration des Endabschnitts 28, die für die Scherkräfte charakteristisch ist, wird vom Fotodetektor 78 detektiert.

Die Membran (Probe) 42 von Fig. 6 hat Öffnungen 80, durch die die zu erfassende Partikel 46 hindurchtreten können. Wenn die Pipette 24 der Vorrichtung 66 über die Membran 42 gerastert wird, werden einerseits die Öffnungen 80 durch Modulation in der Scherkraft detektiert, andererseits werden die Öffnungen durch lokale Konzentrationsänderungen in den erfaßten Partikeln 46 detektiert, und die Partikel können darüber hinaus ortsaufgelöst charakterisiert werden.

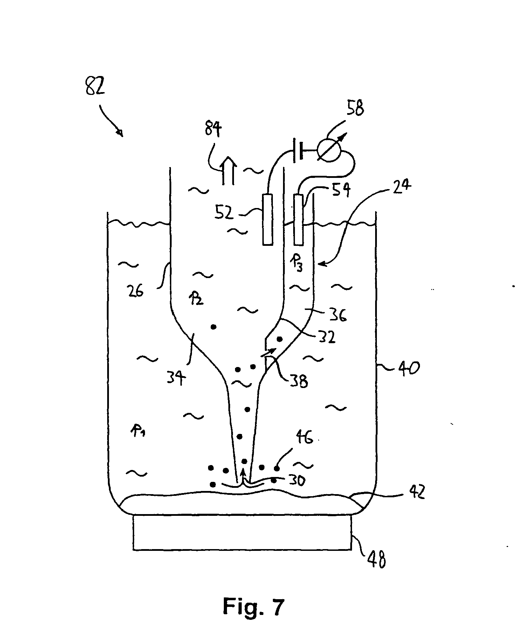

In Fig. 7 ist eine alternative Ausführungsform 82 einer Vorrichtung nach einer Weiterbildung der Erfindung gezeigt. Die Vorrichtung 82 umfaßt eine Pipette 24, die in ihrem Aufbau denjenigen von Fig. 3 und Fig. 6 entspricht. Die Vorrichtung von Fig. 7 umfaßt Mittel (nicht gezeigt) zum Erzeugen eines Druckunterschiedes zwischen dem Druck p₁ im Behälter 40 außerhalb der Pipette 24 und einem Druck p₂ in der ersten Kammer 34 der Pipette 24 wobei p₂ < p₁. Durch diesen Druckunterschied wird auch bei nicht-geladenen zu untersuchenden Partikeln 46 ein Partikelfluß von außerhalb der Pipette 24 durch die Öffnung 30 hindurch in die erste Kammer 34 der Pipette erzeugt. Der Druck p₃ in der zweiten Kammer 36 ist noch geringer als der Druck p₂ in der ersten Kammer 34, so daß zumindest ein Teil des Partikelflusses durch die Nanopore 38 geführt wird.

Der Druckunterschied kann beispielsweise dadurch hergestellt werden, daß an den Kammern 34 und 36 gesaugt wird, wie durch den Pfeil 84 in Fig. 7 schematisch angedeutet ist. Aufgrund des Druckunterschiedes ist der Pegel in der ersten Kammer 34 in der Darstellung von Fig. 7 gegenüber dem Pegel des Elektrolyten in dem Behälter 40 erhöht. Obwohl der Druck p₃ in der zweiten Kammer 36 noch geringer ist als p₂, ist in der Darstellung von Fig. 7 der Elektrolytpegel in der zweiten Kammer 36 nicht sichtbar erhöht, weil der Elektrolyt- und Partikelfluß durch die Nanopore 38 aufgrund deren geringen Querschnitts vergleichsweise gering ist.

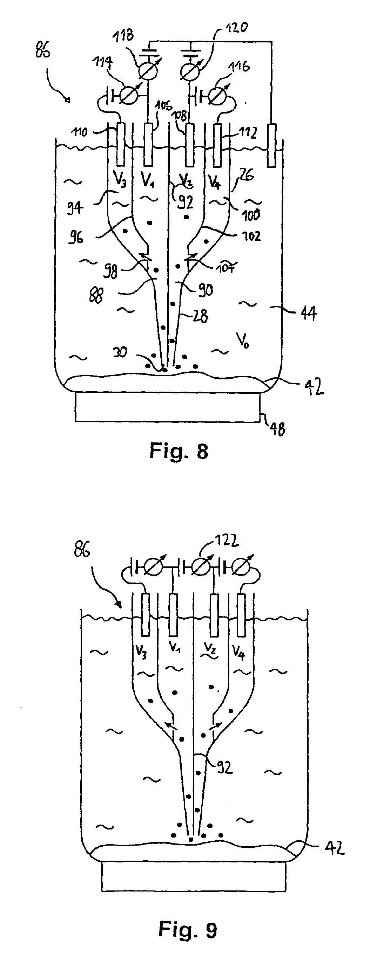

Fig. 8 und 9 zeigen alternative Ausführungsformen der Pipette. Fig. 8 zeigt eine Pipette 86 mit einem Körperabschnitt 26, einem Endabschnitt 28 und einer Öffnung 30. Die Pipette 86 umfaßt eine erste Kammer 88 und eine zweite Kammer 90, die durch eine Trennwand 92 voneinander getrennt sind. Die erste und die zweite Kammer 88, 90 sind beide mit der Öffnung 30 verbunden. Die Pipette 86 umfaßt ferner eine dritte Kammer 94, die von der ersten Kammer 88 durch eine Trennwand 96 getrennt ist, in der eine erste Nanopore 98 ausgebildet ist. Außerdem umfaßt die Pipette 86 eine vierte Kammer 100, die von der zweiten Kammer 90 durch eine Trennwand 102 getrennt ist, in der eine zweite Nanopore 104 ausgebildet ist. In der ersten Kammer 88 ist eine erste Elektrode 106 angeordnet, die die erste Kammer auf einem Potential V₁ hält, in der zweiten Kammer 90 eine zweite Elektrode 108, die die zweite Kammer 90 auf einem Potential V₂ hält, in der dritten Kammer 94 eine dritte Elektrode 110, die die dritte Kammer 94 auf einem Potential V₃ hält und in der vierten Kammer 100 eine vierte Elektrode 112, die die vierte Kammer 100 auf einem Potential V₄ hält. Bezüglich der Potentiale V₁ bis V₄ gilt die folgende Beziehung: V₃ > V₁ > V₂ > V₄. Außerhalb der Pipette 24 ist außerdem eine Badelektrode 114 in den Elektrolyten 44 getaucht, die sich auf einem Potential V₀ befindet, wobei V₁ > V₀ > V₂.

Bei der Pipette 86 von Fig. 8 können zu erfassende Partikel 46 unterschiedlicher Ladung getrennt und separat erfaßt und charakterisiert werden. Negativ geladene Partikel 46 werden durch die Öffnung 30 in die erste Kammer 88 transportiert, und ein Teil von ihnen fließt weiter durch die erste Nanopore 98 in die dritte Kammer 94. Beim Durchtritt durch die erste Nanopore 98 können mit Hilfe einer Strommeßvorrichtung 114 Änderungen der Impedanz der ersten Nanopore 98 gemessen werden, wodurch die Partikel 46 wie oben beschrieben erfaßt und charakterisiert werden können. Auf ähnliche Weise gelangen positive zu erfassende Partikel 46 durch die Öffnung 30 in die zweite Kammer 90 und weiter durch die zweite Nanopore 104 in die vierte Kammer 100, wobei die positiven Partikel mit Hilfe einer Strommeßvorrichtung 116 auf oben beschriebene Weise erfaßt und charakterisiert werden können. Mit Hilfe der Strommeßvorrichtungen 118 und/oder 120 kann ferner der Abstand zwischen dem Endabschnitt 28 der Pipette 86 und der Probe 42 auf die oben beschriebene Weise gemessen werden, so daß auch die Pipette 86 als Rastersonde verwendbar ist.

Fig. 9 zeigt dieselbe Pipette 86 wie Fig. 8, bei der lediglich die Elektroden 106, 108, 110 und 112 anders geschaltet sind. Der Übersichtlichkeit halber sind in Fig. 9 alle Bezugszeichen weggelassen, die mit denjenigen von Fig. 8 identisch sind. Der Hauptunterschied zwischen Fig. 8 und Fig. 9 besteht darin, daß bei der Vorrichtung von Fig. 9 der Strom gemessen wird, der zwischen der ersten Kammer 88 und der zweiten Kammer 90 fließt. Dazu wird eine Strommeßvorrichtung 122 verwendet, die zwischen die erste Elektrode 106 und die zweite Elektrode 108 geschaltet ist. Der Ionenstrom zwischen der ersten Kammer 88 und der zweiten Kammer 90 fließt um das in der Darstellung von Fig. 9 untere Ende der Trennwand 92 herum. Dieser Ionenstrom wird beim Annähern der Pipette 86 an die Probe 42 abgeschnürt, so daß er sich zur Abstandsmessung eignet.

Figuren 8 und 9 zeigen nur beispielhafte Weiterentwicklungen der Pipette, die für die erfindungsgemäße Vorrichtung und das erfindungsgemäße Verfahren verwendbar sind, und viele weitere Modifikationen sind möglich. Beispielsweise könnten die erste und die zweite Nanopore 98, 104 unterschiedliche Größen haben, um die zu erfassenden Partikel 46 nach ihrer Größe zu charakterisieren oder zu sortieren. Auch könnte man sich die Trennwand 92 in Fig. 8 und Fig. 9 wegdenken und sich statt dessen eine Vielzahl von Kammern vorstellen, die jeweils durch eine Nanopore unterschiedlicher Größe mit einer Hauptkammer verbunden sind, die ihrerseits mit der Öffnung 30 verbunden ist.

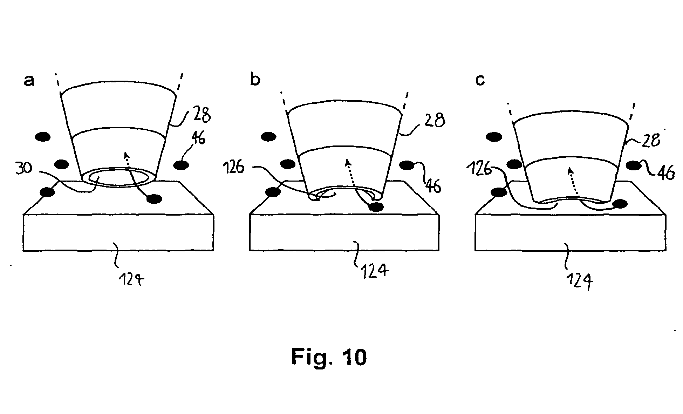

Eine weitere Ausführungsform der vorliegenden Erfindung wird im Zusammenhang mit Fig. 10 beschrieben. Fig. 10 zeigt Momentaufnahmen des äußersten Endes eines Endabschnittes 28 einer Pipette, der in ein nachgiebiges Material 124 gedrückt wird. Bei dem nachgiebigen Material 124 kann es sich beispielsweise um eine Polymermaterialschicht, beispielsweise eine Schicht aus Poly-dimethylsiloxan handeln, die extra zu diesem Zweck bereitgestellt ist. Bei dem nachgiebigen Material 124 kann es sich aber auch um eine Probe handeln, beispielsweise eine Zelle. Der XYZ-Scanner 48 ist dazu ausgelegt, den Endabschnitt 28 der Pipette 24 derart in das nachgiebige Material 124 zu drücken, daß sich die effektive Öffnungsfläche der Öffnung 30 verringert. Diese Verringerung der Öffnungsfläche ist in der Abfolge von Momentaufnahmen a bis c zu erkennen, bei der die effektive Öffnung der Öffnung 30 nach und nach sichelförmig eingeengt wird. Dadurch wird die Öffnung 30 zu einer Nanopore mit veränderbarer Größe.

In dem Beispiel von Fig. 10 ist die Pipette schräg zu der Oberfläche des nachgiebigen Materials gerichtet, wodurch sich die sichelförmige Einschnürung der effektiven Öffnungsfläche ergibt. Die Pipette könnte jedoch auch senkrecht zur Oberfläche gerichtet sein und dafür das Ende der Pipette abgeschrägt sein.

Wie eingangs erwähnt wurde, bildet die Öffnung 30 der Pipette bei den hier im Detail beschriebenen Ausführungsformen selbst nicht die Nanopore für die Erfassung und Charakterisierung von Partikeln nach dem Coulter-Prinzip. Vielmehr ist die Öffnung 30 hier größer, als dies für die Coulter-Messungen zweckdienlich wäre, damit der Widerstand für den Ionenstrom durch den Endabschnitt der Pipette nicht zu groß wird. Statt dessen waren bei den Ausführungsformen von Fig. 3 und Fig. 6 bis 9 die Nanoporen in einer Trennwand im Inneren der Pipette angeordnet.

Die Ausführungsform von Fig. 10 gibt eine Möglichkeit an, die Nanopore gewissermaßen während des Betriebs der Vorrichtung auszubilden, indem die Pipettenöffnung 30 auf die in Fig. 10 gezeigte Weise verengt wird, um dadurch die für die beabsichtigte Messung geeignete Nanopore zu erzeugen. Die Vorrichtung benötigt dann keine weitere Nanopore im Inneren der Pipette mehr.

Beim Betrieb der Vorrichtung nach dieser Weiterbildung kann die Pipette, ähnlich wie im Zusammenhang mit Fig. 3 beschrieben, über eine Probe gerastert werden, wobei der Abschnüreffekt zur Abstandmessung verwendet wird. Dann kann die Pipette zum Zwecke der Partikelmessung weiter auf die Probe zu bewegt werden, um dadurch die effektive Öffnungsfläche der Öffnung 30 zu verringern. Bei derart verringerter effektiver Öffnungsfläche kann die teilweise verdeckte Öffnung 30 als Nanopore 126 für die Coulter-Messung verwendet werden. Dabei kann die effektive Öffnungsfläche der Öffnung 30 variiert werden, um die Partikel noch genauer zu charakterisieren. Die Größe der effektiven Öffnungsfläche kann beispielsweise über die Stärke eines Ionenstroms in Gegenwart eines bekannten Elektrolyten eingestellt werden, der zwischen den Durchgängen von Partikeln durch die Öffnung 30 fließt.

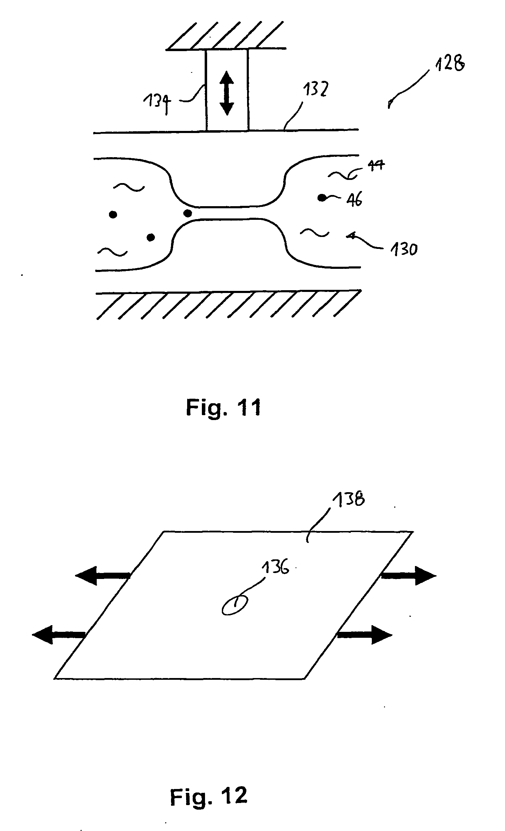

Auch die Nanoporen 38, 98, 104 aus den Figuren 3 und 6 bis 9 können einen veränderbaren Querschnitt haben. Beispiele für Nanoporen mit veränderbarem Querschnitt sind in Fig. 11 und 12 gezeigt. Fig. 11 zeigt eine Nanopore 128, die durch einen Kanal 130 gebildet wird, der in einem flexiblen Material 132 ausgebildet ist. Der Kanal 130 kann beispielsweise mit konventioneller Mikrokontakt-Printing-Technologie hergestellt werden. Ein Piezoaktor 134 ist so angeordnet, daß er das flexible Material 132 zusammendrücken und dadurch den Kanal 130 verengen kann, um dadurch den Querschnitt der Nanopore 128 zu verengen. Wenn der Piezoaktor 134 sich wieder zusammenzieht, weitet sich der Kanal 130 wieder. Somit ist die Verstellung der Größe der Nanopore 128 reversibel. In Fig. 11 deuten die schraffierten Bereiche Abschnitte einer Trennwand oder Membran an, in der die Nanopore 128 angeordnet ist, bei denen es sich beispielsweise um Abschnitte der Trennwand 32 von Fig. 3 handeln könnte.

Fig. 11 zeigt eine Nanopore 136, die in einer flexiblen Membran 138 ausgebildet ist. Durch Zug auf die Membran, wie er durch die Pfeile in Fig. 12 schematisch angedeutet ist, verformt sich die Membran 138, und die Größe der Nanopore 136 verändert sich. Der Zug kann beispielsweise durch Piezoaktoren (nicht gezeigt) bewerkstelligt werden.

## Patentansprüche

1. Vorrichtung (22, 66) zum Erfassen und/oder Charakterisieren von Partikeln (46), die folgendes umfaßt:
mindestens eine Nanopore (38, 98, 104, 126),
eine Spannungsquelle zum Erzeugen eines elektrischen Potentialunterschiedes zwischen den beiden Seiten der mindestens einen Nanopore (38, 98, 104, 126), um einen Ionenstrom durch die Nanopore (38, 98, 104, 126) zu erzeugen, wenn die Nanopore (38, 98, 104, 126) von einem Elektrolyten (44) umgeben ist; und
eine Meßeinrichtung (58, 114, 116), die geeignet ist, eine Änderung der Impedanz der mindestens einen Nanopore (38, 98, 104, 126) bezüglich des Ionenstromes zu erfassen, wenn eines oder mehrere von zu erfassenden Partikeln (46), die in dem Elektrolyten (44) enthalten sind, die mindestens eine Nanopore (38, 98, 104, 126) durchquert bzw. durchqueren,
eine Pipette (24, 86) mit einem Endabschnitt (28), in dem eine Öffnung (30) ausgebildet ist,
Mittel zum Erzeugen eines Partikelflusses von den zu erfassenden Partikeln (46) von außerhalb der Pipette (24, 86) durch die Öffnung (30) und durch die Nanopore (38, 98, 104, 126); **gekennzeichnet durch**
einen XYZ-Scanner (48) zum Bewegen der Pipette (24, 86) relativ zu einer Probe (42).

2. Vorrichtung (22, 66) nach Anspruch 1, bei der die mindestens eine Nanopore (38, 98, 104, 126) eine veränderbare Größe hat.

3. Vorrichtung (22, 66) nach Anspruch 2, bei der die mindestens eine Nanopore (38, 98, 104, 126) durch einen Kanal in einem flexiblen Material gebildet wird, dessen Querschnitt durch Druck auf das flexible Material verengbar ist, oder bei der die mindestens eine Nanopore (38, 98, 104, 126) in einer Membran angeordnet ist und der Querschnitt der Nanopore durch Dehnung der Membran veränderbar ist.

4. Vorrichtung nach Anspruch 2, mit einem Probenbehälter (40) zum Aufnehmen des Elektrolyten (44) und mit einem XYZ-Scanner (48) zum Bewegen der Pipette (24, 86) und des Probenbehälters (40) relativ zueinander, wobei der XYZ-Scanner (48) dazu ausgelegt ist, den Endabschnitt (28) der Pipette derart in ein der Öffnung (30) im Endabschnitt (28) gegenüberliegendes nachgiebiges Material (124) zu drücken, daß sich die effektive Öffnungsfläche der Öffnung (30) verringert, um **dadurch** die Nanopore (126) veränderbarer Größe zu bilden.

5. Vorrichtung (22, 66) nach Anspruch 1, mit einem Probenbehälter (40) zum Aufnehmen des Elektrolyten (44) und mit einer Steuerungseinrichtung (62) zum Ansteuern des XYZ-Scanners (48) zum Abtasten der Oberfläche einer Probe (42), indem der Endabschnitt (28) der Pipette (24, 86) in einem konstanten Abstand über die Oberfläche der Probe (42) gefahren wird.

6. Vorrichtung (22, 66) nach Anspruch 5, mit einer Datenverarbeitungseinrichtung (62), die so programmiert ist, daß sie die beim Abtasten der Oberfläche der Probe (42) durchgeführten Relativbewegungen zwischen dem Endabschnitt (28) der Pipette (24, 86) und der Probe (42) aufzeichnet und daraus ein topographisches Bild der Probe (42) und/oder ein Bild erzeugt, das die Messungen der Partikel (46) in Abhängigkeit vom Ort der Pipettenöffnung (30) repräsentiert.

7. Vorrichtung (22, 66) nach Anspruch 5 oder 6, bei der der Abstand zwischen dem Endabschnitt (28) der Pipette (24, 86) und der Probe (42) basierend auf einem Ionenstrom durch die Öffnung (30) im Endabschnitt (28) der Pipette (24, 86), oder basierend auf Scherkräften eingestellt wird, die auf den Endabschnitt (28) der Pipette (24, 86) wirken.

8. Vorrichtung (22, 66) nach einem der vorhergehenden Ansprüche, bei der der Innenraum der Pipette (24, 86) eine erste Kammer (34, 88) hat, die direkt mit der Öffnung (30) im Endabschnitt (28) der Pipette (24, 86) verbunden ist, und eine zweite Kammer (36, 94), die von der ersten Kammer (34, 88) durch eine Trennwand (32, 96) getrennt ist, in welcher die mindestens eine Nanopore (38, 98) ausgebildet ist.

9. Vorrichtung nach Anspruch 8, bei der der Innenraum der Pipette (24, 86) eine dritte Kammer (100) enthält, die von der ersten Kammer (90) durch eine Trennwand (102) getrennt ist, in welcher mindestens eine Nanopore (104) ausgebildet ist.

10. Vorrichtung nach Anspruch 9, bei der die Nanopore (98) in der Trennwand (96) zwischen der ersten Kammer (88) und der zweiten Kammer (94) und die Nanopore (104) in der Trennwand (102) zwischen der ersten Kammer (90) und der dritten Kammer (100) eine unterschiedliche Öffnungsfläche haben.

11. Vorrichtung nach Anspruch 9 oder 10, bei der die Differenz zwischen den elektrischen Potentialen der zweiten Kammer und der ersten Kammer ein anderes Vorzeichen hat als die Differenz zwischen den elektrischen Potentialen der dritten Kammer und der ersten Kammer.

12. Verfahren zum Erfassen und/oder Charakterisieren von Partikeln, bei dem mindestens eine Nanopore (38, 98, 104, 126) in einem Elektrolyten (44) angeordnet wird, in welchem zu detektierende Partikel (46) enthalten sind;
ein elektrischer Potentialunterschied zwischen den beiden Seiten der Nanopore (38, 98, 104, 126) hergestellt wird, um einen Ionenstrom durch die Nanopore (38, 98, 104, 126) zu erzeugen;
Änderungen der Impedanz der mindestens einen Nanopore (38, 98, 104, 126) bezüglich des Ionenstroms erfaßt werden, wenn eines oder mehrere der zu erfassenden Partikel (46) die mindestens eine Nanopore (38, 98, 104, 126) durchquert bzw. durchqueren; und
ein Partikelfluß von den zu detektierenden Partikeln (46) von außerhalb einer Pipette (24, 86) durch eine Öffnung (30) in einem Endabschnitt (28) der Pipette (24, 86) und durch die Nanopore (38, 98, 104, 126) hindurch erzeugt wird, **dadurch gekennzeichnet, daß** die Pipette (24, 86) mithilfe eines XYZ-Scanners relativ zu einer Probe (46) bewegt wird, um die genannten Partikel ortsaufgelöst zu erfassen und/oder zu charakterisieren.

13. Verfahren nach Anspruch 12, bei dem die Größe der mindestens einen Nanopore (38, 98, 104, 126) verändert wird, insbesondere indem der Endabschnitt (28) der Pipette derart in ein der Öffnung (30) im Endabschnitt (28) gegenüberliegendes nachgiebiges Material (124) gedrückt wird, daß sich die effektive Öffnungsfläche der Öffnung (30) verringert, um **dadurch** eine Nanopore (126) veränderbarer Größe zu bilden.

14. Verfahren nach Anspruch 12 oder 13, bei dem der Endabschnitt (28) der Pipette (24, 86) in einem konstanten Abstand über die Oberfläche einer Probe (42) gefahren wird und die beim Abtasten der Oberfläche der Probe (42) durchgeführten Relativbewegungen zwischen dem Endabschnitt (28) der Pipette (24, 86) und der Probe (42) aufgezeichnet werden und daraus ein topographisches Bild der Probe (42) und/oder ein Bild erzeugt wird, das die Messungen der Partikel (46) in Abhängigkeit vom Ort der Pipettenöffnung (30) repräsentiert.

15. Verfahren nach Anspruch 14, bei dem der Abstand zwischen dem Endabschnitt (28) der Pipette (24, 86) und der Probe (42) basierend auf einem Ionenstrom durch die Öffnung (30) im Endabschnitt (28) der Pipette (24, 86) oder basierend auf Scherkräften eingestellt wird, die auf den Endabschnitt (28) der Pipette (24, 86) wirken.

## Claims

1. A device (22, 66) for detecting and/or characterising particles (46) comprising:
at least one nanopore (38, 98, 104, 126),
a voltage source for generating an electrical potential between the two sides of the at least one nanopore (38, 98, 104, 126) in order to produce an ion current through the nanopore (38, 98, 104, 126) when the nanopore (38, 98, 104, 126) is surrounded by an electrolyte (44); and
a measuring device (58, 114, 116) that is suitable for detecting a change in the impedance of the at least one nanopore (38, 98, 104, 126) with respect to the ion current when one or more of the particles (46) to be detected contained in the electrolyte (44) pass(es) the at least one nanopore (38, 98, 104, 126),
a pipette (24, 86) having an end section (28) in which an opening (30) is formed,
means for producing a particle flow of the particles (46) to be detected from outside the pipette (24, 86) through the opening (30) and through the nanopore (38, 98, 104, 126); **characterized by**
an XYZ-scanner (48) for moving the pipette (24, 86) relative to a sample (42).

2. The device (22, 66) of claim 1 in which the at least one nanopore (38, 98, 104, 126) is of variable size.

3. The device (22, 66) of claim 2 in which the at least one nanopore (38, 98, 104, 126) is formed by a channel in a flexible material the cross section of which can be narrowed by applying pressure to the flexible material or in which the at least one nanopore (38, 98, 104, 126) is arranged in a membrane and the cross-section of the nanopore can be changed by stretching the membrane.

4. The device (22, 66) of claim 2, further comprising a sample container (40) for containing the electrolyte (44) and with an XYZ-scanner (48) for moving the pipette (24, 86) and the sample container (40) relative to each other, wherein the XYZ-scanner (48) is suitable for pressing the end section (28) of the pipette into a compliant material (124) facing the opening (30) in the end section (28) in such a way that the effective opening area of the opening (30) is reduced in order to thereby form said nanopore (126) of variable size.

5. The device (22, 66) of claim 1, said device comprising a sample container (40) and a control system (62) for controlling the XYZ scanner (48) for scanning the surface of a sample (42) moving the end section (28) of the pipette (24, 86) at a constant distance across the surface of the sample (42).

6. The device (22, 66) of claim 5, further comprising a dataprocessing system (62) which is programmed in such a way that the relative movements between the end section (28) of the pipette (24, 86) and the sample (42) carried out during scanning of the surface of the sample (42) are recorded and a topographical image of the sample (42) is produced therefrom and/or an image is produced that represents the measurements of the particles (46) as a function of the location of the pipette opening (30).

7. The device (22, 66) of claims 5 or 6 in which the distance between the end section (28) of the pipette (24, 86) and the sample (42) is adjusted based on an ion current through the opening (30) in the end section (28) of the pipette (24, 86) or based on shear forces that act on the end section (28) of the pipette (24, 86).

8. The device (22, 66) of any one of the preceding claims in which the interior of the pipette (24, 86) has a first chamber (34, 88) which is directly connected to the opening (30) in the end section (28) of the pipette (24, 86), and a second chamber (36, 94) which is separated from the first chamber (34, 88) by a partition wall (32, 96) in which the at least one nanopore (38, 98) is formed.

9. The device (22, 66) of claim 8 in which the interior of the pipette (24, 86) comprises a third chamber (100) which is separated from the first chamber (90) by a partition wall (102) in which at least one nanopore (104) is formed.

10. The device of claim 9 in which the nanopore (98) in the partition wall (96) between the first chamber (88) and the second chamber (94) and the nanopore (104) in the partition wall (102) between the first chamber (90) and the third chamber (100) have different opening areas.

11. The device of claim 9 or 10 in which the difference between the electrical potentials of the second chamber and the first chamber has a sign opposite to that of the difference between the electrical potentials of the third chamber and the first chamber.

12. A method of detecting and/or characterising particles, comprising the steps of:
arranging at least one nanopore (38, 98, 104, 126) in an electrolyte (44) containing particles (46) to be detected;
generating an electrical potential difference between the two sides of the nanopore (38, 98, 104, 126) in order to produce an ion current through the nanopore (38, 98, 104, 126); and
detecting changes in the impedance of the at least one nanopore (38, 98, 104, 126) with regard to the ion current when one or more of the particles (46) to be detected pass(es) through the at least one nanopore (38, 98, 104, 126);
generating a particle flow of particles (46) to be detected from outside a pipette (24, 86) through an opening (30) in an end section (28) of the pipette (24, 86) and through the nanopore (38, 98, 104, 126), **characterized in that**
the pipette (24, 86) is moved relative to a sample (46) by means of an XYZ-scanner in order to detect and/or characterise the particles in a location resolved manner.

13. The method of claim 12 in which the size of the at least one nanopore (38, 98, 104, 126) is changed, in particular by
pressing the end section (28) of the pipette into a compliant material (124) lying opposite the opening (30) in the end section (28) in such a way that the effective opening area of the opening (30) is reduced in order to thereby produce a nanopore (126) of variable size.

14. The method of any one of claims 12 or 13 in which the end section (28) of the pipette (24, 86) is moved at a constant distance across the surface of a sample (42) and the relative movements between the end section (28) of the pipette (24, 86) and the sample (42) carried out during the scanning of the surface of the sample (42) are recorded and used to produce a topographical image of the sample (42) and/or an image representing the measurements of the particles (46) as a function of the location of the pipette opening (30).

15. The method of claim 14 in which the distance between the end section (28) of the pipette (24, 86) and the sample (42) is adjusted based on an ion current through the opening (30) in the end section (28) of the pipette (24, 86) or based on shear forces acting on the end section (28) of the pipette (24, 86).

## Revendications

1. Dispositif (22, 66) pour la détection et/ou la caractérisation de particules (46), lequel comprend les éléments suivants :
au moins un nanopore (38, 98, 104, 126), une source de tension pour la génération d'une différence de potentiel électrique entre les deux côtés du ou des nanopores (38, 98, 104, 126), pour produire un courant ionique au travers du nanopore (38, 98, 104, 126) quand le nanopore (38, 98, 104, 126) est entouré d'un électrolyte (44) ; et
un module de mesure (58, 114, 116) servant à détecter une variation de l'impédance du ou des nanopores (38, 98, 104, 126) par rapport au courant ionique, quand une ou plusieurs des particules à détecter (46), contenues dans l'électrolyte (44), traversent le ou les nanopores (38, 98, 104, 126),
une pipette (24, 86) avec une section d'extrémité (28) où est ménagée une ouverture (30),
des moyens pour produire un flux des particules à détecter (46) depuis l'extérieur de la pipette (24, 86), traversant l'ouverture (30) et le nanopore (38, 98, 104, 126) ; **caractérisé par**
un scanner XYZ (48) pour déplacer la pipette (24, 86) par rapport à un échantillon (42).

2. Dispositif (22, 66) selon la revendication 1, où le ou les nanopores (38, 98, 104, 126) ont une grandeur variable.

3. Dispositif (22, 66) selon la revendication 2, où le ou les nanopores (38, 98, 104, 126) sont formés par un canal dans un matériau flexible, dont la section peut être rétrécie par pression sur le matériau flexible, ou bien où le ou les nanopores (38, 98, 104, 126) sont ménagés dans une membrane et où la section du nanopore est variable par dilatation de la membrane.

4. Dispositif selon la revendication 2, avec un conteneur à échantillons (40) pour recevoir l'électrolyte (44) et avec un scanner XYZ (48) pour déplacer la pipette (24, 86) et le conteneur à échantillons (40) l'une par rapport à l'autre, où le scanner XYZ (48) est prévu pour pousser la section d'extrémité (28) de la pipette dans un matériau souple (124) opposé à l'ouverture (30) de la section d'extrémité (28), de manière à restreindre la surface d'ouverture effective de l'ouverture (30) pour former ainsi le nanopore (126) de grandeur variable.

5. Dispositif (22, 66) selon la revendication 1, avec un conteneur à échantillons (40) pour recevoir l'électrolyte (44) et avec un module de commande (62) pour la commande de balayage par le scanner XYZ (48) de la surface d'un échantillon (42), la section d'extrémité (28) de la pipette (24, 86) étant déplacée à intervalle constant au-dessus de la surface de l'échantillon (42).

6. Dispositif (22, 66) selon la revendication 5, avec un module de traitement des données (62), lequel est programmé de manière à enregistrer les déplacements relatifs effectués entre la section d'extrémité (28) de la pipette (24, 86) et l'échantillon (42) lors du balayage de la surface de l'échantillon (42) et à en générer une image topographique de l'échantillon (42) et/ou une image représentant les mesures des particules (46) en fonction de l'emplacement de l'ouverture de pipette (30).

7. Dispositif (22, 66) selon la revendication 5 ou la revendication 6, où l'intervalle entre la section d'extrémité (28) de la pipette (24, 86) et l'échantillon (42) est réglé sur la base d'un courant ionique traversant l'ouverture (30) de la section d'extrémité (28) de la pipette (24, 86), ou sur la base des efforts de cisaillement exercés sur la section d'extrémité (28) de la pipette (24, 86).

8. Dispositif (22, 66) selon l'une des revendications précédentes, où l'intérieur de la pipette (24, 86) contient un premier compartiment (34, 88), lequel est directement relié à l'ouverture (30) de la section d'extrémité (28) de la pipette (24, 86), et un deuxième compartiment (36, 94), lequel est séparé du premier compartiment (34, 88) par une cloison de séparation (32, 96) où le ou les nanopores (38, 98) sont formés.

9. Dispositif selon la revendication 8, où l'intérieur de la pipette (24, 86) contient un troisième compartiment (100), lequel est séparé du premier compartiment (90) par une cloison de séparation (102) où au moins un nanopore (104) est formé.

10. Dispositif selon la revendication 9, où le nanopore (98) dans la cloison de séparation (96) entre le premier compartiment (88) et le deuxième compartiment (94), et le nanopore (104) dans la cloison de séparation (102) entre le premier compartiment (90) et le troisième compartiment (100) sont de surfaces d'ouverture différentes.

11. Dispositif selon la revendication 9 ou la revendication 10, où la différence entre les potentiels électriques du deuxième compartiment et du premier compartiment est affectée d'un autre signe algébrique que la différence entre les potentiels électriques du troisième compartiment et du premier compartiment.

12. Procédé pour la détection et/ou la caractérisation de particules, où au moins un nanopore (38, 98, 104, 126) est disposé dans un électrolyte (44) contenant les particules à détecter (46) ;
une différence de potentiel électrique est produite entre les deux côtés du nanopore (38, 98, 104, 126), pour générer un courant ionique traversant le nanopore (38, 98, 104, 126) ;
des variations de l'impédance du ou des nanopores (38, 98, 104, 126) par rapport au courant ionique sont détectées quand une ou plusieurs des particules à détecter (46) traversent le ou les nanopores (38, 98, 104, 126) ; et où un flux des particules à détecter (46) est produit depuis l'extérieur d'une pipette (24, 86), traversant une ouverture (30) dans une section d'extrémité (28) de la pipette (24, 86) et le nanopore (38, 98, 104, 126), **caractérisé en ce que** la pipette (24, 86) est déplacée par rapport à un échantillon (46) au moyen d'un scanner XYZ, pour détecter et/ou pour caractériser en résolution locale lesdites particules.

13. Procédé selon la revendication 12, où la grandeur du ou des nanopores (38, 98, 104, 126) est variée, notamment en poussant la section d'extrémité (28) de la pipette dans un matériau souple (124) opposé à l'ouverture (30) de la section d'extrémité (28), de manière à restreindre la surface d'ouverture effective de l'ouverture (30) pour former ainsi un nanopore (126) de grandeur variable.

14. Procédé selon la revendication 12 ou 13, où la section d'extrémité (28) de la pipette (24, 86) est déplacée à intervalle constant au-dessus de la surface d'un échantillon, et où les déplacements relatifs effectués entre la section d'extrémité (28) de la pipette (24, 86) et l'échantillon (42) lors du balayage de la surface de l'échantillon (42) sont enregistrés, une image topographique de l'échantillon (42) et/ou une image représentant les mesures des particules (46) en fonction de l'emplacement de l'ouverture de pipette (30) en étant générées.

15. Procédé selon la revendication 14, où l'intervalle entre la section d'extrémité (28) de la pipette (24, 86) et l'échantillon (42) est réglé sur la base d'un courant ionique traversant l'ouverture (30) de la section d'extrémité (28) de la pipette (24, 86), ou sur la base des efforts de cisaillement exercés sur la section d'extrémité (28) de la pipette (24, 86).
